# EUROPEAN PATENT SPECIFICATION

(11) **EP 0 638 545 B1**
(45) Date of publication and mention of the grant of the patent: **13.10.1999**
(21) Application number: 94305506.1
(22) Date of filing: 26.07.1994
(51) Int. Cl.: C07C 241/04, C07C 243/38

(54) **Process for preparing 1,2-diacyl-2-t-alkylhydrazides**
Verfahren zur Herstellung von 1,2-Diacyl-2-t-alkylhydroziden
Procédé de préparation de 1,2-diacyl-2-alkylhydrazides

(30) Priority: 11.08.1993 US 105083
(43) Date of publication of application: 15.02.1995
(73) Proprietor: ROHM AND HAAS COMPANY, Philadelphia, Pennsylvania 19106-2399 (US)
(72) Inventor: Kelly, Martha Jean, Norristown, Pennsylvania 19403 (US)
(74) Representative: Tanner, James Percival

(56) References cited:
- EP-A- 0 286 746
- EP-A- 0 492 839

## Description

The present invention is concerned with a process for preparing a 1, 2-diacyl-2-tertiary alkylhydrazide. More particularly, the present invention is concerned with a process for preparing the aforesaid hydrazide wherein, in a first step, an aromatic acid anhydride is reacted with a defined hydrazine so that the resultant intermediate is obtained in the substantial absence of undesirable isomers. In a second step, the resultant mono acyl hydrazide is reacted with an aromatic acid chloride to obtain the final product. These hydrazides are known to have insecticidal activity against Coleoptera and Lepidoptera.

It is known to prepare a 1, 2-diacyl-2-t-alkylhydrazide by reacting an aromatic benzoyl chloride with a tertiary alkyl hydrazine to yield a mono acyl hydrazide. Thereafter, the hydrazide may be reacted with a second aromatic acid chloride to yield the desired diacyl hydrazide.

When the aromatic acid chloride is reacted with the t-alkyl hydrazine to yield the intermediate hydrazide, the reaction results in two possible mono acylated products and one diacylated impurity. The desired intermediate product, however, is an acyl-2-alkylhydrazide. The mono acylated side product reacts in the second step of the process to form a new impurity, and the original diacylated impurity is carried through the process unchanged. Both side products provide contaminants of the final desired product. It therefore becomes necessary to remove the undesired side products in order to obtain a substantially pure 1, 2-diacyl-2-t-alkylhydrazide.

"1-Acetyl-1-alkyl- and 1-acetyl-2-alkylhydrazines", S.S.Kirov Military-Medical Academy, translated from Zhurnal Organicheskoi Khimii, Volume 13, Number 8, pages 1585-1589, Zelenin, et al August, 1977 (original article submitted June 9, 1976) discloses the reaction of a monoalkylhydrazine with acetic anhydride.

"The Synthesis and Reactions of Sterically Hindered Hydrazines; And The Acid-Catalyzed Rearrangement of Tertiary Cyclic Azides", Lakritz J., University of Michigan, Ph.D. (1960) discloses the reaction of t-butylhydrazine with acetic anhydride and adding an excess of benzoyl chloride to obtain N'-acetyl-N-benzoyl-N-t-butylhydrazine.

Neither reference teaches the reaction of a t-alkylhydrazine with an aromatic acid anhydride to prepare a monoacyl hydrazide and the enhanced selectivity of the reaction for the desired product.

EP-A-0286746 discloses various processes to N-(optionally substituted)-N'-substituted-N,N'-disubstituted hydrazines. The processes therein include reaction of various acyl chlorides, esters or mixed anhydrides with substituted hydrazines in which the equivalent R² substituent is different from that of the present invention. There are also disclosed various processes in which the substituted hydrazine is reacted with a ketone to form a hydrazone or with an organic carbonate to form an acyloxyhydrazide intermediate. None of the processes herein teach or suggest the reaction of a t-alkylhydrazine with an aromatic acid anhydride to prepare a monoacyl hydrazide and the enhanced selectivity of the reaction for the desired product.

The present invention enables a process to be provided for the production of a diacyltertiary alkylhydrazide wherein the presence of by-products is substantially reduced or eliminated. The present invention also enables there to be provided a process, as aforesaid, wherein the process is at an economic advantage due to the substantial absence or reduction of by-products and subsequent yield losses due to the necessary removal of such by-products. The present invention further enables there to be provided the aforesaid process wherein an aromatic carboxylic acid by-product is recycled in the process.

According to the present invention there is provided a process for preparing a 1,2-diacyl-2-t-alkyl hydrazide, of the formula: 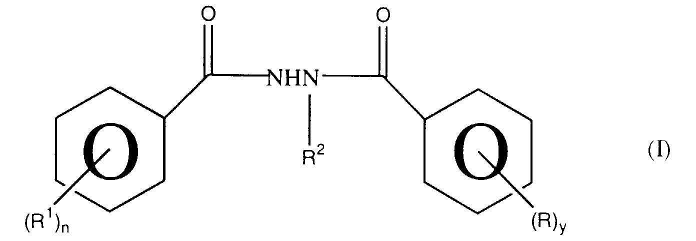 wherein R is different from R¹, n is 0 or an integer of from 1 to 5, and y is 0 or an integer from 1 to 5, which comprises the steps of:-
(i) reacting, at a temperature of 0 to 90°C, an aromatic acid anhydride with a hydrazine, the aromatic acid anhydride being neat or in an organic solvent and having the formula: 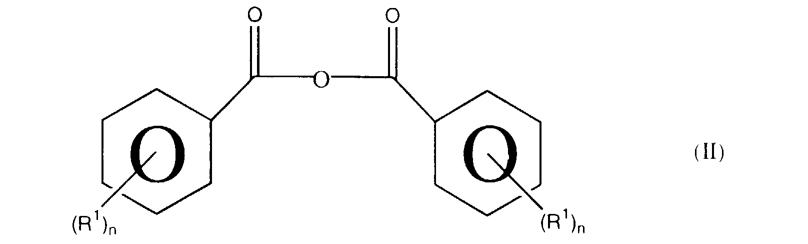 wherein n is 0 or an integer of from 1 to 5, each R¹ is independently selected from the group consisting of halo, alkyl, alkoxy, haloalkyl and haloalkoxy, and the hydrazine being dissolved in water and having the formula:

   R²NHNH₂ (III)

   wherein R² is selected from the class consisting of C4-C8 tertiary alkyl groups;
   to produce an aqueous phase and an organic phase, the organic phase containing a mono acyl hydrazide of the formula 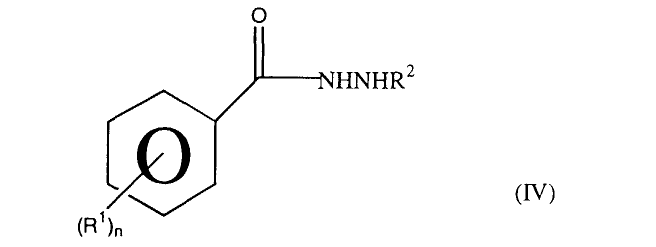 and the aqueous phase containing an aromatic carboxylic acid having the formula: 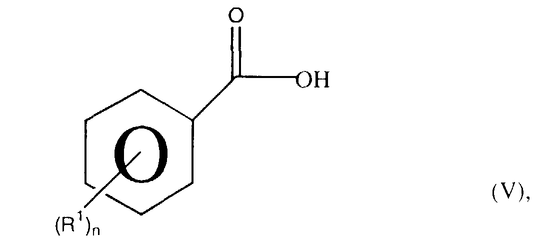 the reaction product being substantially free of 1-acyl-1-t-alkyl hydrazide;
(ii) reacting, in an organic solvent and at a temperature of 0 to 95°C, the mono acyl hydrazide of formula (IV), prepared in step (i), with an aromatic acid chloride having the formula: 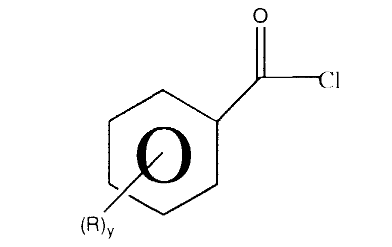 wherein y is 0 or an integer of from 1 to 5 and each R is independently selected from the group consisting of halo, alkyl, alkoxy, haloalkyl and haloalkoxy, to produce a 1,2-diacyl-2-t-alkyl hydrazide of formula (I).

As used herein:-
Alkyl includes straight or branched alkyl groups such as (C₁-C₆)alkyl, for example methyl, ethyl, n-propyl, n-butyl, isopropyl, t-butyl or neopentyl;
Alkoxy includes (C₁-C₆)alkoxy such as methoxy, ethoxy or butoxy;
Halo means bromo, chloro, fluoro or iodo;
Haloalkyl includes halo(C₁-C₆)alkyl such as trifluoromethyl, chloroethyl, trichloromethyl or pentafluoroethyl;
Haloalkoxy includes halo(C₁-C₆)alkoxy such as trifluoromethoxy.

It is preferred however, that R¹ be hydrogen, 4-ethyl, 4-chloro or (2-methyl-3-methoxy).

Among the aromatic acid anhydrides which may be used in practicing this invention are benzoic acid anhydride, 4-ethylbenzoic anhydride, 4-chlorobenzoic anhydride and 2-methyl-3-methoxybenzoic anhydride.

The hydrazine which may be used in practicing the first step of the process will have the formula R²NHNH₂. In this formula, R² is a C-4 to C-8 tertiary alkyl group, such as tertiary butyl, 1,1-dimethylpentyl or 1,1,3,3-tetramethylbutyl. Among the hydrazines which may be specifically mentioned are: t-butylhydrazine, 1,1-dimethylpentylhydrazine and 1,1,3,3-tetramethylbutylhydrazine.

The reaction product from the first step is reacted with an aromatic acid chloride having the formula: 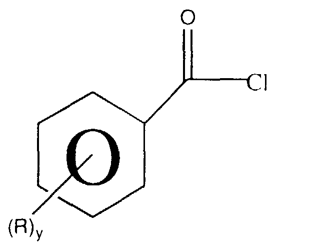 y is 0 or an integer from 1 to 5 and in this formula, R is selected from the same substituents as is R¹. However, for a particular diacyl hydrazide, R is different from R¹. Preferably, "n" is 0 or "(R₁)ₙ" is 3,5-dimethyl.

Particularly preferred aromatic acid chlorides are benzoyl chloride and 3,5-dimethylbenzoyl chloride.

The first step of the process of the invention may be conducted under an atmosphere of nitrogen and at ambient pressure. In the first step, the free t-alkyl hydrazine may be obtained by adding a base to a solution of the hydrazine salt, such as the hydrochloride. The reaction of the hydrazine with the anhydride may be conducted in a solvent, such as metylene chloride, xylene, toluene, ethyl acetate, n-butyl acetate or diethyl ether. The reaction may be conducted at a temperature of from 0°C to 90°C. The reaction preferably continues until the anhydride is consumed. Thereafter, the aqueous and organic solvent phases of the reaction mixture may be separated and the aqueous phase may be washed with an organic solvent to recover product which also may be present in the aqueous phase. The solvent phases may be stripped on a rotary evaporator or other suitable equipment. The product phase may then be analyzed by HPLC (high performance liquid chromatography) or other standard techniques. The material obtained is of high purity. Alternatively, the solution of the product in organic solvent may be used directly in the second step without isolation. The aromatic carboxylic acid, which is obtained as a by-product, is in the aqueous phase. The acid may be recovered by acidification followed by extraction or filtration. The acid may then be converted to the anhydride for subsequent reuse in the process.

The amount of reactants used in the first step is preferably a slight excess of the acid anhydride over stoichiometry.

The second step of the process, the reaction of a mono acyl hydrazide with an aromatic acid chloride, may be conducted under a nitrogen atmosphere. The mono acyl hydrazide may be dissolved in an organic solvent, such as toluene. Water may also be present. An equivalent of acid chloride and an equivalent of base may be added to the hydrazide. The reaction may be conducted at a temperature of from 0°C to 95°C. When the reaction is complete, the product may be isolated either by stripping solvent from the washed organic phase or by crystallizing the product from the reaction mixture by allowing it to cool followed by filtration of the product from the liquid. The organic phase may then be washed with a suitable organic solvent such as toluene.

In one aspect of the process of this invention, a branched C4-C8 alkylhydrazine is dissolved in water. If a t-alkyl hydrazine salt is used, an equivalent of base is added to neutralize the salt to the hydrazine.

An aromatic acid anhydride is added, either neat or in an organic solvent, and the temperature maintained at from 0°C to 90°C and preferably between 0°C and 25°C. After the addition is completed, the reaction mixture is stirred at a low temperature and then allowed to warm to room temperature and held there until the reaction is complete as evidenced by the disappearance of anhydride. The phases are separated into an organic and aqueous phase and the organic phase is washed with dilute base and/or water. The organic phase contains the mono acylhydrazide and the solvent is removed. Alternatively, the intermediate is not isolated but is used directly in the second step of the process. The carboxylic acid by-product, which is in the aqueous phase, may be recovered by acidification followed by extraction or filtration. The recovered acid may then be converted to the anhydride for subsequent reuse in the process. The resultant mono acylhydrazide is then reacted with an aromatic acid chloride at a temperature of from 0°C to 95°C to yield the final product.

The final product, obtained in the process of the present invention, may be sufficiently pure to use without further purification. If further purification is desired, it may be accomplished by crystallizing the product from the reaction mixture by cooling, by recrystallization of the isolated product, by chromatography or by any other standard technique.

The purity of the final product (the diacyl t-alkyl hydrazide) may be determined by standard techniques such as gas chromatography, high performance liquid chromatography, and nuclear magnetic resonance spectroscopy.

The following Examples are presented to illustrate various embodiments of the present invention. In the following Examples, all reactions are conducted under an atmosphere of nitrogen. The reported yields are corrected for analytical purity.

### Example 1 - 2-t-butyl-1-benzoylhydrazide

This Example illustrates the high degree of selectivity when practicing the process of this invention.

In a round bottom flask equipped with a stirrer, a two phase solution of 40 mmol of t-butylhydrazine was prepared by adding 4 mL of 10 M sodium hydroxide solution to 4.98 g of t-butylhydrazine hydrochloride in 30 mL of methylene chloride. The reaction mixture was cooled, then treated simultaneously with a solution of benzoic anhydride (9.50 g, 42 mmol) in methylene chloride, and 4 mL of 10 M sodium hydroxide solution. The temperature was maintained at 10°C. At the end of the addition, 20 mL of DI water was added, the reaction mixture is stirred for two hours at low temperature, then warmed to room temperature and held there for 1.5 hours. The phases were separated. The aqueous phase was washed with methylene chloride. The combined methylene chloride phases were dried over sodium sulfate, filtered, stripped on a rotary evaporator and dried under vacuum to give 7.23 g (94% yield) of the 2-t-butyl-1-benzoylhydrazide. The analysis by high performance liquid chromatography (HPLC) revealed >99% purity of the material. The isomer 1-t-butyl-1-benzoyl hydrazide was present at a level of 0.4%, and <0.1% of the diacylated hydrazide was detected.

### Example 2: Preparation of N'-tert-butyl-N-(4-ethylbenzoyl)hydrazide

This Example illustrates the recovery of p-ethylbenzoic acid which may then be converted to the anhydride for reuse in practicing the process of this invention.

t-Butylhydrazine hydrochloride (12.47 g, 100 mmol), 40 g of water, 9.0 g of 50% sodium hydroxide and 60 mL n-butylacetate were combined in a flask. p-Ethylbenzoic anhydride (28.32 g, 100 mmol) and an aqueous solution of potassium carbonate (13.92 g, 101 mmol) were co-fed to the reaction mixture, which was maintained at a temperature of 20°C. At the end of the reaction, the product was a slurry. Additional n-butyl acetate (60 mL) was added, and the reaction mixture was heated to 35°C to dissolve the solids. The aqueous phase was removed. The organic phase was washed twice with 10% potassium carbonate solution and twice with distilled water, then stripped on a rotary evaporator and dried under vacuum. The product was 20.69 g, which was analyzed by HPLC as 100% 2-t-butyl-1-(4-ethylbenzoyl)hydrazide. The yield was 94%. The combined aqueous phases were acidified to pH 1 with hydrochloric acid then extracted three times with methylene chloride. The combined methylene chloride phases were stripped and dried under vacuum to give 15.54 g (104 mmol) of p-ethylbenzoic acid.

### Example 3 - 2-t-butyl-1-(4-ethylbenzoyl)hydrazide

This Example illustrates the selectivity of the process of this invention for the desired 1,2-diacyl-2-t-alkyl hydrazide and an in situ conversion of p-ethylbenzoic acid to the anhydride for use in the process of this invention.

To a 300 mL round bottom flask equipped with a stirrer was added 23.65 g of p-ethylbenzoic acid (0.1575 mol) and 50 mL of water. Sodium hydroxide solution (50%, 12.0 g, 0.150 mol) and 4.9 g of 40% tetra-n-butylammonium hydroxide (0.0075 mol) were added and the reaction mixture was stirred until all the acid dissolved. The reaction mixture was cooled to 5°C, and 26.26 g of p-ethylbenzoyl chloride (96.3% purity, 0.150 mol) in 40 mL of methylene chloride was added over 10 minutes. The reaction mixture was held at 5°C for one hour, then a solution of 19.07 g of t-butylhydrazine hydrochloride (0.150 mol) in 50 mL of water was added over 5 minutes. The reaction temperature was raised to 15°C, then 24 g of 50% sodium hydroxide solution (0.300 mol) was added over 15 minutes. The reaction mixture was held at 15°C for a few hours, then worked up. The phases were separated, the aqueous phases were washed twice with methylene chloride. The combined organic phase was dried over magnesium sulfate and stripped on a rotary evaporator to give a solid which was dried under vacuum. The product, 2-t-butyl-1(4-ethylbenzoyl)hydrazide weighed 33.08 g. The yield was 95% of material with a purity of 94.7%. The isomer and the diacylated material were each present at <0.5%.

### Examples 4 though 6

The procedure of Example 2 was generally repeated except that the following changes were made as indicated in Table 1. Non-critical variations in the amount of solvent used and presence of drying agent were present.

**TABLE 1**

| **Ex. No.** | **Anhydride Used** | **Solvent Used** | **Reaction Temperature Used** | **Base Used** | **Yield** | **Ratio of A:B:C**** |
|---|---|---|---|---|---|---|
| 4 | p-toluic | Methylene chloride | 5°C to room temperature | NaOH | 96% | 98.8:<0.1:0.2 |
| 5 | p-EBAA* | Toluene | 20°C | NaOH and K₂CO₃ | 94% | 100:<0.1:<0.4 |
| 6 | p-EBAA* | Toluene | 15°C | NaOH | 91% | 94:<0.1:<0.4 |

| | | | | | | |
|---|---|---|---|---|---|---|
| * p-EBAA is p-ethylbenzoic anhydride. | | | | | | |
| ** A=2-t-butyl-1-acylhydrazide, B=1-t-butyl-1-acylhydrazide, C=diacylhydrazide. | | | | | | |

### Example 7 (comparative)-2-t-butyl-1-(4-ethylbenzoyl)hydrazide

The purpose of this Example is to demonstrate the reduced selectivity obtained when preparing an acyl hydrazide by a prior art method using a benzoyl chloride instead of an aromatic anhydride.

### Reaction of t-butylhydrazine with an acid chloride

The reaction was run in a round bottom flask equipped with an overhead stirrer under an atmosphere of nitrogen. A solution of t-butylhydrazine hydrochloride (26.25g, 0.210 mol) and 33.60 g of 50% sodium hydroxide solution (0.42 mol) in 46 mL of water was cooled to 15°C. Methylene chloride (265 mL) was added. 4-Ethylbenzoyl chloride (33.73 g, 97.7% purity, 0.195 mol) was added dropwise over 1 hour to the reaction mixture, the temperature of which was maintained at 15°C. The reaction mixture was held for 15 minutes after the addition was complete, then was warmed to room temperature. The phases were separated. The aqueous phase was washed with 50 mL of methylene chloride. The combined methylene chloride phases were washed with 95 mL of water, then stripped on a rotary evaporator. The product was dried in a vacuum oven at 50°C to yield 43.23 g of a white solid. The analysis of the solid (by HPLC) was 91.3% 2-t-butyl-1-(4-ethylbenzoyl)hydrazide, 6.1% 1-t-butyl-1-(4-ethylbenzoyl)hydrazide and 1.4% 1-t-butyl-1,2-bis-(4-ethylbenzoyl)hydrazide. The yield of 2-t-butyl-1-(4-ethylbenzoyl)hydrazide was 92% based on the starting acid chloride.

## Claims

1. A process for preparing a 1,2-diacyl-2-t-alkyl hydrazide, of the formula: 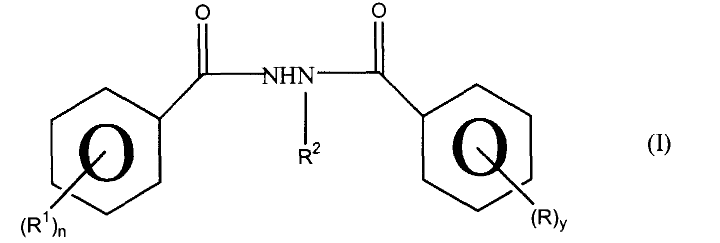 wherein R is different from R¹, n is 0 or an integer of from 1 to 5, and y is 0 or an integer from 1 to 5, which comprises the steps of:-
(i) reacting, at a temperature of 0 to 90°C, an aromatic acid anhydride with a hydrazine, the aromatic acid anhydride being neat or in an organic solvent and having the formula: 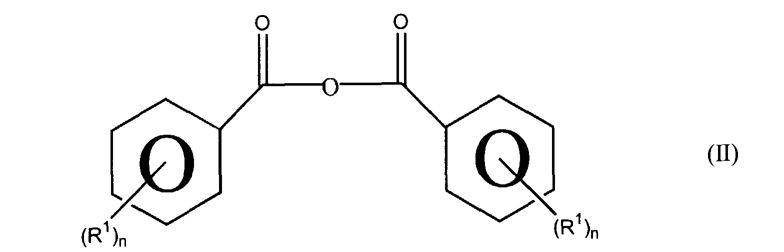 wherein n is 0 or an integer of from 1 to 5, each R¹ is independently selected from the group consisting of halo, alkyl, alkoxy, haloalkyl and haloalkoxy, and the hydrazine being dissolved in water and having the formula:
R²NHNH₂ (III)
wherein R² is selected from the class consisting of C₄-C₈ tertiary alkyl groups;
to produce an aqueous phase and an organic phase, the organic phase containing a mono acyl hydrazide of the formula 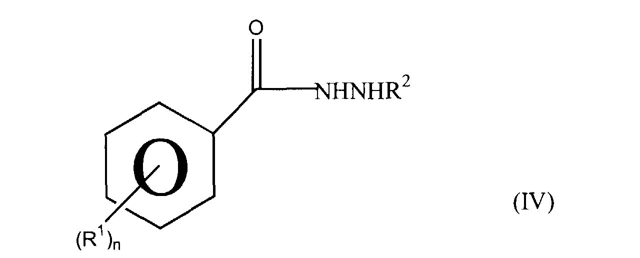 and the aqueous phase containing an aromatic carboxylic acid having the formula: 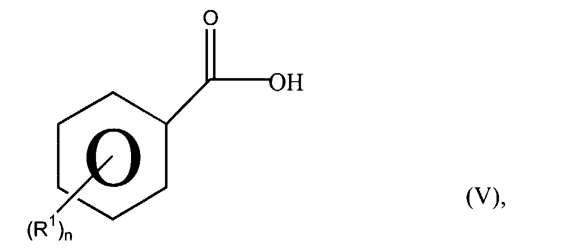 the reaction product being substantially free of 1-acyl-1-t-alkyl hydrazide;
(ii) reacting, in an organic solvent and at a temperature of 0 to 95°C, the mono acyl hydrazide of formula (IV), prepared in step (i), with an aromatic acid chloride having the formula: 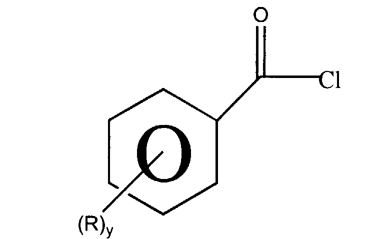 wherein y is 0 or an integer of from 1 to 5 and each R is independently selected from the group consisting of halo, alkyl, alkoxy, haloalkyl and haloalkoxy, to produce a 1,2-diacyl-2-t-alkyl hydrazide of formula (I).

2. A process as claimed in claim 1, wherein said aromatic acid anhydride is selected from the group consisting of benzoic anhydride, 4-ethylbenzoic anhydride, 4-chlorobenzoic anhydride and 2-methyl-3-methoxybenzoic anhydride, and is preferably 4-ethylbenzoic anhydride or benzoic anhydride.

3. A process as claimed in claim 1 or claim 2, wherein said tertiary alkyl group of said hydrazine is selected from the group consisting of tertiary butyl, 1,1-dimethylpentyl and 1,1,3,3-tetramethylbutyl, and preferably said hydrazine is t-butylhydrazine.

4. A process as claimed in claim 1, wherein said mono-acyl hydrazide is selected from the group consisting of 2-t-butyl-1-(4-ethylbenzoyl)hydrazide, 2-t-butyl-1-benzoylhydrazide, 2-t-butyl-1-(4-chlorobenzoyl)hydrazide, and 2-t-butyl-1-(2-methyl-3-methoxybenzoyl)hydrazide.

5. A process as claimed in claim 1, wherein said aromatic carboxylic acid is selected from the group consisting of benzoic acid, 4-ethylbenzoic acid, 4-chlorobenzoic acid, and 2-methyl-3-methoxybenzoic acid.

6. A process as claimed in any preceeding claim, wherein the reaction of the acid anhydride with the hydrazine is conducted in the presence of an organic solvent.

7. A process as claimed in any preceding claim, wherein both of said reactions are conducted in the presence of an organic solvent selected from the group consisting of xylene, toluene, methylene chloride, ethyl acetate, n-butyl acetate, isopropyl acetate and isobutyl acetate.

8. A process as claimed in any preceding claim, wherein said aromatic carboxylic acid is converted to an anhydride and is subsequently used in the process as the aromatic acid anhydride.

## Patentansprüche

1. Verfahren zur Herstellung eines 1,2-Diacyl-2-t-alkylhydrazids der Formel 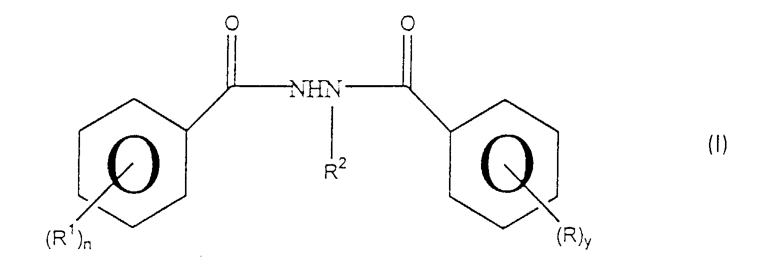 wobei R unterschiedlich von R¹ ist, n 0 oder eine ganze Zahl von 1 bis 5 ist und y 0 oder eine ganze Zahl von 1 bis 5 ist, welches die Schritte
(i) des Umsetzens eines aromatischen Säureanhydrids mit einem Hydrazin bei einer Temperatur von 0 bis 90°C, wobei das aromatische Säureanhydrid in Reinform oder in einem organischen Lösungsmittel vorliegt und die Formel 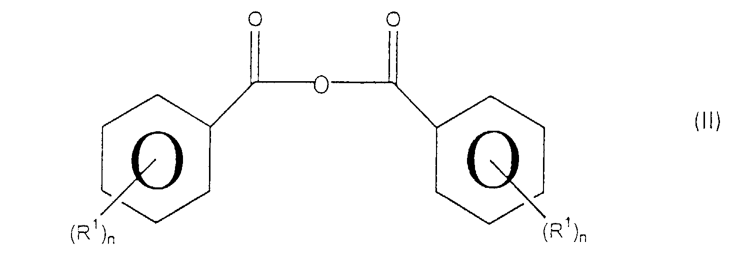 aufweist, wobei n 0 oder eine ganze Zahl von 1 bis 5 ist, jedes R¹ unabhängig voneinander ausgewählt ist aus der Gruppe, bestehend aus Halogen, Alkyl, Alkoxy, Haloalkyl und Haloalkoxy, und wobei das Hydrazin in Wasser gelöst wird und die Formel
R²NHNH₂ (III)
aufweist, wobei R² ausgewählt ist aus der Klasse, bestehend aus C₄-C₈ -tertiären Alkylresten, um eine wässerige Phase und eine organische Phase herzustellen, wobei die organische Phase ein Monoacylhydrazid der Formel 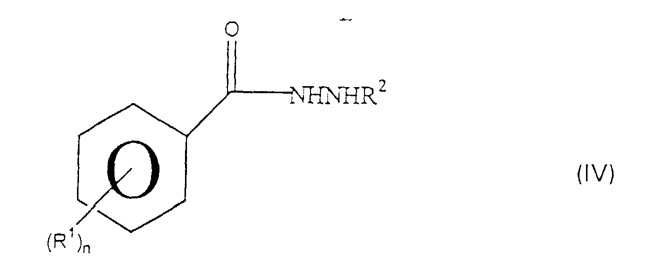 enthält, und wobei die wässerige Phase eine aromatische Carbonsäure mit der Formel 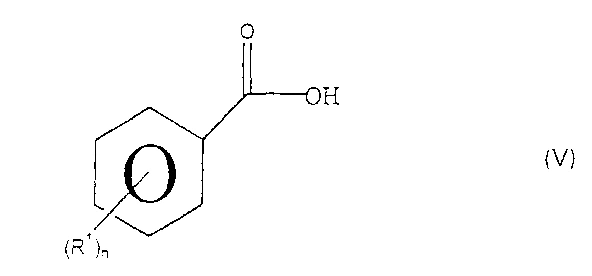 enthält, wobei das Reaktionsprodukt im wesentlichen frei von 1-Acyl-1-t-alkylhydrazid ist, und
(ii) des Umsetzens des Monoacylhydrazids der Formel (Vl), hergestellt in Schritt (i) mit einem aromatischen Säurechlorid mit der Formel 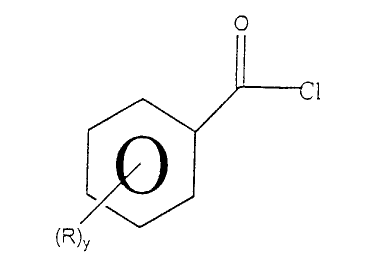 wobei y 0 oder eine ganze Zahl von 1 bis 5 ist, und jedes R unabhängig voneinander ausgewählt ist aus der Gruppe, bestehend aus Halogen, Alkyl, Alkoxy, Haloalkyl und Haloalkoxy, in einem organischen Lösungsmittel und bei einer Temperatur von 0 bis 95°C zur Herstellung eines 1,2-Diacyl-2-t-alkylhydrazids der Formel (1), umfaßt.

2. Verfahren nach Anspruch 1, wobei das aromatische Säureanhydrid ausgewählt ist aus der Gruppe, bestehend aus Benzoesäureanhydrid, 4-Ethylbenzoesäureanhydrid, 4-Chlorbenzoesäureanhydrid und 2-Methyl-2-methoxybenzoesäureanhydrid, und vorzugsweise 4-Ethylbenzoesäureanhydrid oder Benzoesäureanhydrid ist.

3. Verfahren nach Anspruch 1 oder 2, wobei der tertiäre Alkylrest des Hydrazins ausgewählt ist aus der Gruppe, bestehend aus tertiär Butyl, 1,1-Dimethylpentyl und 1,1,3,3-Tetramethylbutyl, und vorzugsweise das Hydrazin t-Butylhydrazin ist.

4. Verfahren nach Anspruch 1, wobei das Monoacylhydrazid ausgewählt ist aus der Gruppe, bestehend aus 2-t-Butyl-1-(4-ethylbenzoyl)hydrazid, 2-t-Butyl-1-benzoylhydrazid, 2-t-Butyl-1-(4-chlorbenzoyl)hydrazid und 2-t-Butyl-1-(2-methyl-3-methoxybenzoyl)hydrazid.

5. Verfahren nach Anspruch 1, wobei die aromatische Carbonsäure ausgewählt ist aus der Gruppe, bestehend aus Benzoesäure, 4-Ethylbenzoesäure, 4-Chlorbenzoesäure und 2-Methyl-3-methoxybenzoesäure.

6. Verfahren nach einem der vorhergehenden Ansprüche, wobei die Umsetzung des Säureanhydrids mit dem Hydrazin in Gegenwart eines organischen Lösungsmittels durchgeführt wird.

7. Verfahren nach einem der vorhergehenden Ansprüche, worin beide Umsetzungen in Gegenwart eines organischen Lösungsmittels, ausgewählt aus der Gruppe, bestehend aus Xylol, Toluol, Methylenchlorid, Ethylacetat, n-Butylacetat, lsopropylacetat und lsobutylacetat, durchgeführt werden.

8. Verfahren nach einem der vorhergehenden Ansprüche, worin die aromatische Carbonsäure in ein Anhydrid umgewandelt wird und anschließend in dem Verfahren als aromatisches Säureanhydrid verwendet wird.

## Revendications

1. Procédé de préparation d'un 1,2-diacyl-2-tert-alkylhydrazide de formule: 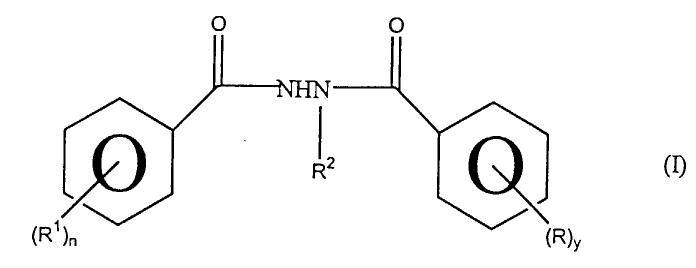 dans laquelle R est différent de R¹, n vaut 0 ou est un entier de 1 à 5, et y vaut 0 ou est un entier de 1 à 5, qui comprend les étapes de :
(i) réaction, à une température de 0 à 90°C, d'un anhydride d'acide aromatique avec une hydrazine, l'anhydride d'acide aromatique étant exempt de solvant ou se trouvant dans un solvant organique et ayant la formule 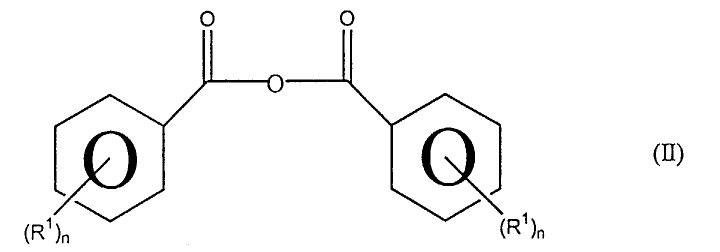 dans laquelle n vaut 0 ou est un entier de 1 à 5, chaque radical R¹ est choisi indépendamment des autres dans l'ensemble comprenant les groupes halogéno, alkyle, alcoxy, halogènalkyle et halogènalcoxy, l'hydrazine étant dissoute dans de l'eau et ayant la formule :
R²NHNH₂ (III)
dans laquelle R² est choisi dans l'ensemble comprenant les groupes alkyle tertiaires en C₄-C₈ ;
pour produire une phase aqueuse et une phase organique, la phase organique contenant un monoacylhydrazide de formule 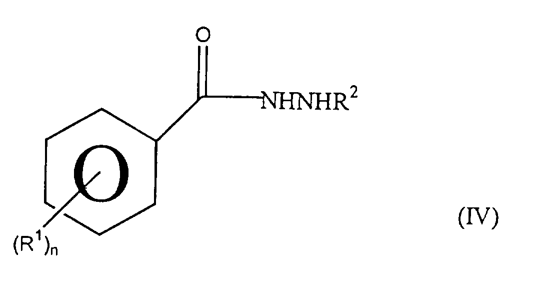 et la phase aqueuse contenant un acide carboxylique aromatique ayant la formule : 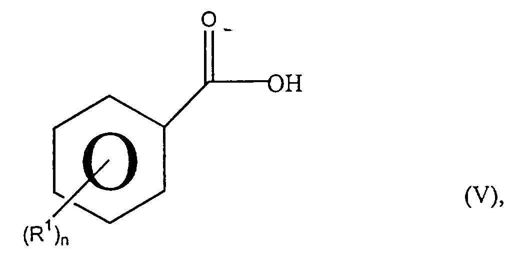 le produit de la réaction étant essentiellement exempt de 1-acyl-1-tert-alkylhydrazide ;
(ii) réaction, dans un solvent organique et à une température de 0 à 95°C, du monoacylhydrazide de formule (IV) préparé dans l'étape (i) avec un chlorure d'acide aromatique ayant la formule 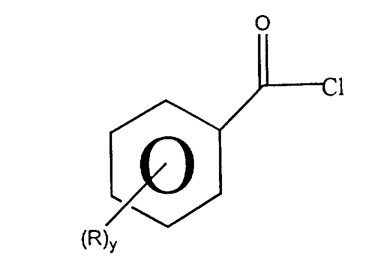 dans laquelle y vaut 0 ou est un entier de 1 à 5, et chaque radical R est choisi indépendamment des autres dans l'ensemble comprenant les groupes halogéno, alkyle, alcoxy, halogènalkyle et halogènalcoxy, pour produire un 1,2-diacyl-2-tert-alkylhydrazide de formule (I).

2. Procédé selon la revendication 1, dans lequel ledit anhydride d'acide aromatique est choisi dans l'ensemble comprenant l'anhydride benzoïque, l'anhydride 4-éthylbenzoïque, l'anhydride 4-chlorobenzoïque et l'anhydride 2-méthyl-3-méthoxybenzoïque, et est de préférence l'anhydride 4-éthylbenzoïque ou l'anhydride benzoïque.

3. Procédé selon la revendication 1 ou 2, dans lequel ledit groupe alkyle tertiaire de ladite hydrazine est choisi dans l'ensemble comprenant les groupes tert-butyle, 1,1-diméthylpentyle et 1,1,3,3-tétraméthylbutyle, et de préférence ladite hydrazine est la tert-butylhydrazine.

4. Procédé selon la revendication 1, dans lequel ledit monoacylhydrazide est choisi dans l'ensemble comprenant le 2-tert-butyl-1-(4-éthylbenzoyl)hydrazide, le 2-tert-butyl-1-benzoylhydrazide, le 2-tert-butyl-1-(4-chlorobenzoyl)hydrazide et le 2-tert-butyl-t-(2-méthyl-3-méthoxybenzoyl)hydrazide.

5. Procédé selon la revendication 1, dans lequel ledit acide carboxylique aromatique est choisi dans l'ensemble comprenant l'acide benzoïque, l'acide 4-éthylbenzoïque, l'acide 4-chlorobenzoïque et l'acide 2-méthyl-3-méthoxybenzoïque.

6. Procédé selon l'une quelconque des revendications précédentes, dans lequel la réaction de l'anhydride d'acide avec l'hydrazine est mise en oeuvre en présence d'un solvant organique.

7. Procédé selon l'une quelconque des revendications précédentes, dans lequel les deux réactions sont mises en oeuvre en présence d'un solvant organique choisi dans l'ensemble comprenant le xylène, le toluène, le chlorure de méthylène, l'acétate d'éthyle, l'acétate de n-butyle, l'acétate d'isopropyle et l'acétate d'isobutyle.

8. Procédé selon l'une quelconque des revendications précédentes, dans lequel ledit acide carboxylique aromatique est converti en un anhydride, puis est utilisé dans le procédé en tant qu'anhydride d'acide aromatique.
